# FASCICULE DE BREVET EUROPEEN

(11) **EP 3 560 366 B1**
(45) Date de publication et mention de la délivrance du brevet: **06.09.2023**
(21) Numéro de dépôt: 19169235.9
(22) Date de dépôt: 15.04.2019
(51) Int. Cl.: A41D 31/32, B32B 5/24, A41D 13/00, A61N 5/06

(54) **VÊTEMENT COMPRENANT UN REVÊTEMENT RÉFLECTEUR**
KLEIDUNGSSTÜCK, BESTEHEND AUS EINER REFLEKTORBESCHICHTUNG
GARMENT COMPRISING A REFLECTING COATING

(30) Priorité: 25.04.2018 FR 1800443
(43) Date de publication de la demande: 30.10.2019
(73) Titulaire: Salomon S.A.S., 74370 Metz-Tessy (FR)
(72) Inventeur: CHAPUIS, Serge, 73610 Lepin Le Lac (FR); TORTEL, Arnaud, 26220 Dieulefit (FR)
(74) Mandataire: Lapierre, Stéphane

(56) Documents cités:
- EP-A1- 3 031 431
- CN-A- 106 334 270
- FR-A1- 2 957 495
- JP-A- 2003 180 794

## Description

La présente invention concerne un vêtement comprenant une base textile munie d'un revêtement réflecteur constitué d'au moins un matériau absorbant et restituant des rayonnements infrarouges lointains (IRL).

Un matériau comprenant au moins un oxyde métallique, notamment un oxyde métallique minéral, constitue un tel matériau. Lorsqu'il est positionné, en vis-à-vis du corps d'un individu, ce type de matériau produit des effets bénéfiques et, notamment, contribue à stimuler la circulation sanguine.

Un des effets bénéfiques consiste à provoquer une sensation de chaleur au niveau de la peau (épiderme et/ou derme) où l'énergie des rayonnements infrarouges lointains restitués est convertie en chaleur.

C'est ce que vise le document US 9719206 en proposant de recouvrir des parties de vêtement avec une couche interne comprenant de la céramique, dans le but de garder la chaleur émise par le corps. Pour obtenir cette sensation de chaleur escomptée, la couche interne couvre, de manière régulière, une grande surface du panneau de tissu. La couche interne peut couvrir entièrement une partie substantielle de la surface interne du vêtement. Alternativement, la couche interne reproduit un motif dont le revêtement en céramique couvre, de manière régulière, entre 10 et 90% d'une partie du panneau de tissu. Selon une variante, le vêtement proposé peut comprendre des portions localisées, sans couche interne, afin d'améliorer la respirabilité du vêtement, comme par exemple, au niveau des aisselles.

L'utilisation de matériau à base d'oxydes métalliques, absorbant et restituant des rayonnements infrarouges lointains (IRL), permet également d'autres effets bénéfiques comme :
- augmenter le flux sanguin au niveau de la peau grâce à la dilatation des vaisseaux capillaires, ce qui favorise la circulation sanguine,
- activer le métabolisme,
- diminuer les sensations de douleur.

Pour que ces effets bénéfiques soient optimaux, il est important de maintenir le matériau absorbant et restituant des rayonnements infrarouges lointains au plus près de la peau d'un individu, à des emplacements déterminés choisis.

Certains fabricants proposent des patchs « céramiques » destinés à venir se coller directement sur la peau pour couvrir des zones précises et déterminées. Ces positionnements particuliers correspondent à une cartographie précise qui conditionne l'efficacité de l'utilisation de ces patchs. Ces localisations peuvent être par exemple :
- des carrefours vasculo-nerveux périphériques;
- des points particuliers du muscle afin d'augmenter l'effet de contraction ou de détente;
- des points particuliers du tendon afin d'augmenter la détente, la pliométrie, la proprioception;
- des points particuliers nerveux (cerveau moelle épinière, plexus nerveux du ventre) afin d'avoir des effets sur le coeur, la détente globale, l'équilibre, les systèmes ortho et parasympathique, le psychisme.

Ces patchs constituent ainsi un moyen pratique pour stimuler précisément des points particuliers du corps. Cependant, pour obtenir des effets bénéfiques optimaux, l'utilisateur doit connaître précisément la cartographie des zones à stimuler et s'en rappeler, ce qui n'est pas évident. Ensuite, il doit coller les patchs au bon endroit. Cette opération n'est pas non plus évidente, notamment dans des parties difficilement accessibles, seul, comme par exemple, le dos. Enfin, coller un patch directement sur la peau présente un autre inconvénient au moment où il faut le retirer. En effet, cette opération peut blesser l'utilisateur du fait que la colle ait très probablement collé des poils.

CN 106 334 270 décrit un vêtement selon le préambule de la revendication 1.

Le but de l'invention est de proposer un vêtement muni d'un revêtement réflecteur permettant de renvoyer des rayonnements IRL vers des parties ciblées du corps de l'utilisateur.

Un but est notamment de faciliter la mise en position de revêtements réflecteurs dans des zones précises et déterminées.

Un autre but est de proposer un vêtement dont l'emplacement des zones de revêtement réflecteur composées d'au moins un oxyde métallique est défini en fonction de l'usage du vêtement et de l'effet bénéfique recherché.

Un autre but est de proposer un article vestimentaire ne blessant pas l'utilisateur à la mise en place, ni au retrait, de l'article vestimentaire.

Un autre but est d'améliorer la proprioception des effets induits de ce type de matériau. L'invention propose un vêtement comprenant :
- une base textile destinée à recouvrir une partie du corps de l'utilisateur, la base textile comportant une surface interne destinée à être positionnée en vis-à-vis du corps de l'utilisateur et une surface externe, opposée à la surface interne et,
- un revêtement réflecteur constitué d'un matériau comprenant au moins un oxyde métallique, le revêtement réflecteur recouvrant ou formant une partie de la surface interne de la base textile.

L'article est caractérisé par le fait que le vêtement comprend plusieurs zones ciblées distinctes, chaque zone ciblée étant définie par un disque, de diamètre au moins égal à 1,5 centimètre, à l'intérieur duquel la base textile est recouverte ou formée du revêtement réflecteur sur 90% de la surface interne du disque, chaque zone ciblée étant entourée d'une zone périphérique formant un anneau délimité par un diamètre interne égal au diamètre externe dudit disque et par un diamètre externe d'un cercle égal à quatorze centimètres, dans la zone périphérique, la base textile étant recouverte ou formée du revêtement réflecteur entre 0 et 50% de la surface interne de l'anneau et, par le fait que la totalité des revêtements réflecteurs associés à la surface interne de la base textile recouvre ou forme entre 5% et 50% de la totalité de la surface interne de la base textile, et en ce que au moins une zone ciblée munie du revêtement réflecteur est positionnée sur le vêtement de sorte qu'elle recouvre sensiblement, lorsque le vêtement est porté, un des emplacements spécifiques du corps suivant :
- une zone ciblée haute localisée en vis-à-vis de la partie haute externe du muscle du grand pectoral, sous la clavicule;
- une zone ciblée haute localisée en vis-à-vis du processus ou appendice xiphoïde du sternum;
- une zone ciblée haute localisée en vis-à-vis de l'estomac à mi-distance entre l'appendice xiphoïde et le nombril;
- une zone ciblée haute localisée en vis-à-vis d'une zone bordée par la partie du muscle trapèze supérieur, par le muscle supra-épineux et par le muscle élévateur de la scapula;
- une zone ciblée haute localisée en vis-à-vis de deux zones dorsales situées de part et d'autre des vertèbres dorsales ou thoraciques D11, D12
- une zone ciblée haute localisée en vis-à-vis de deux zones dorsales situées de part et d'autre des vertèbres dorsales ou thoraciques D7, D8;
- une zone ciblée haute localisée en vis-à-vis de deux zones dorsales situées de part et d'autre des vertèbres dorsales ou thoraciques D4, D5.

Cette construction permet de positionner des portions de recouvrement réflecteur au niveau de zones précises et de les isoler par des zones périphériques pour lesquelles le recouvrement réflecteur est moins important. Ainsi, grâce à cette concentration ponctuelle de ce type de matériau à base d'au moins un oxyde métallique, on améliore la restitution des rayonnements infrarouges de ce matériau sur le corps au niveau du point ciblé. Ainsi, le corps ne va pas être perturbé par des effets parasites résultants de la présence de ce type de matériau autour de la zone ciblée. La stimulation du corps est accentuée pour des effets bénéfiques plus marqués. La proprioception des effets de ce type de matériau est également améliorée. D'autre part, l'utilisateur n'a pas à se soucier du positionnement des portions de recouvrement réflecteur selon une quelconque cartographie du corps humain. En effet, il suffit seulement d'enfiler le vêtement pour que les portions de recouvrement réflecteur soient bien positionnées par rapport au corps dans l'objectif d'atteindre l'effet recherché. Enfin, le maintien en position des portions de recouvrement réflecteur est assuré par la tenue du vêtement, cela permet de s'affranchir de toute colle, ce qui améliore fortement le confort de l'utilisateur.

Selon des aspects avantageux mais non obligatoires de l'invention, un tel vêtement peut incorporer une ou plusieurs des caractéristiques suivantes, prises dans toute combinaison techniquement admissible :
- le diamètre externe du disque définissant plusieurs zones ciblées est inférieur à huit centimètres,
- dans la zone périphérique, un revêtement réflecteur est présent et recouvre ou forme une partie de la surface interne de la base textile, au niveau de la surface interne, dans une moindre concentration par rapport à celle de la zone ciblée,
- la zone périphérique de plusieurs zones ciblées comprend une zone libre formant un anneau délimité par un cercle de diamètre égal à onze centimètres et par le cercle de diamètre égal à quatorze centimètres, dans la zone libre, aucun revêtement réflecteur ne couvre ou ne forme une partie de la surface interne de la base textile,
- la zone périphérique comprend un secteur intermédiaire couvrant un anneau délimité par un diamètre interne égal au diamètre dudit disque et par un diamètre externe d'un cercle égal à onze centimètres et dans lequel un revêtement réflecteur est présent et recouvre ou forme une partie de la surface interne de la base textile, au niveau de la surface interne, dans une proportion inférieure à 50% de la surface interne de l'anneau formant le secteur intermédiare,
- la couche de revêtement réflecteur recouvre entièrement la base textile sur au moins une partie dense de la zone ciblée, cette partie dense étant définie par un disque dont le diamètre est au moins égal à 1,5 centimètre,
- plusieurs zones ciblées sont reliées par des zones de liaison munies de revêtement réflecteur,
- plusieurs revêtements réflecteurs sont associés avec la base textile par impression, collage, enduction, tissage, tricotage ou broderie,
- le revêtement réflecteur est ménagé sur des pièces distinctes destinées à être assemblées de manière amovible sur la base textile,
- la base textile est un textile tricoté, tissé ou non-tissé,
- le revêtement réflecteur forme une spirale ou une combinaison de spirales,
- le revêtement réflecteur forme une surcouche dont l'épaisseur est inférieure à un millimètre,
- le revêtement réflecteur comprend au moins un des oxydes métalliques minéraux suivants : Al₂O₃, Fe₂O₃, TiO₂, Cr₂O₃, SiO₂. MgO, ZrO₂, MnO₂, Co₂O₃, Y₂O₃ et leurs mélanges.

D'autres caractéristiques et avantages de l'invention seront mieux compris à l'aide de la description qui va suivre, en regard des dessins annexés illustrant, selon des formes de réalisation non limitatives, comment l'invention peut être réalisée, et dans lequel :
- la figure 1 représente un premier exemple de la répartition du revêtement réflecteur au niveau d'une zone ciblée conformément à l'invention ;
- la figure 2 représente un deuxième exemple de la répartition du revêtement réflecteur au niveau d'une zone ciblée conformément à l'invention ;
- la figure 3 est une vue en coupe du vêtement selon un premier mode de réalisation de l'invention ;
- la figure 4 est une vue en coupe du vêtement selon un deuxième mode de réalisation de l'invention ;
- la figure 5 est une vue antérieure d'un premier vêtement selon l'invention ;
- la figure 6 est une vue postérieure du premier vêtement de la figure 5 ;
- la figure 7 est une vue postérieure d'un deuxième vêtement de la figure selon l'invention ;
- la figure 8 est une vue antérieure d'un troisième vêtement selon un exemple ne formant pas partie de l'invention;
- la figure 9 est une vue postérieure du troisième vêtement de la figure 8 ;

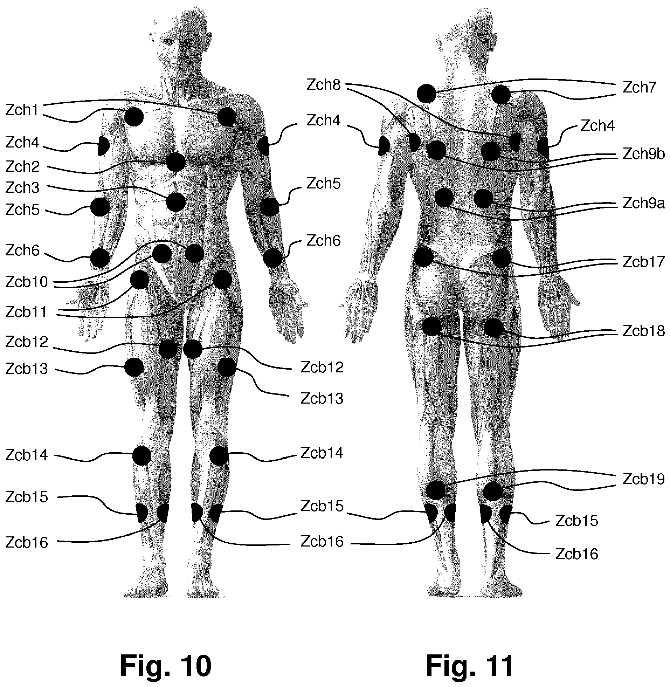

- la figure 10 est une vue antérieure de l'anatomie musculaire d'un corps humain permettant d'identifier la localisation des zones ciblées ;
- la figure 11 est une vue postérieure de l'anatomie musculaire d'un corps humain permettant d'identifier la localisation des zones ciblées ;

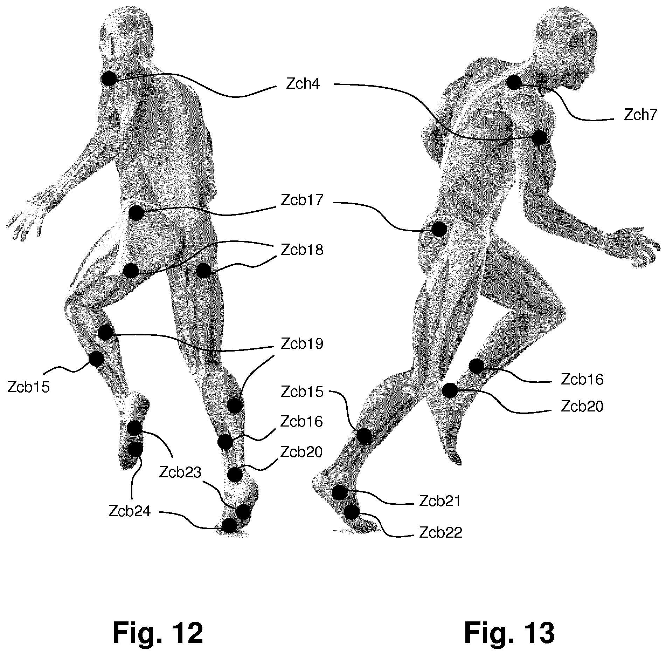

- la figure 12 est une vue ¾ arrière de l'anatomie musculaire d'un corps humain permettant d'identifier la localisation des zones ciblées ;
- la figure 13 est une vue latérale de l'anatomie musculaire d'un corps humain permettant d'identifier la localisation des zones ciblées ;
- la figure 14 est une vue postérieure du squelette d'un corps humain permettant d'identifier la localisation des zones ciblées.

L'invention porte sur un vêtement 1 comprenant une base textile 2 destinée à recouvrir une partie du corps de l'utilisateur. La partie recouverte peut être la partie supérieure du corps. Par exemple, le vêtement peut être un débardeur, un tee-shirt à manches courtes ou à manches longues. Selon un exemple ne formant pas partie de l'invention, la partie recouverte peut être la partie inférieure du corps. Par exemple, le vêtement peut être un short, un corsaire, un pantalon, un collant ou une chaussette. La base textile 2 comporte une surface interne 21 destinée à être positionnée en vis-à-vis du corps de l'utilisateur et une surface externe 22, opposée à la surface interne.

La base textile 2 est un textile tricoté (maille), tissé (tissu chaîne et mailles) ou non-tissé (feutres). Elle est constituée de fibres, filaments ou fils 23 d'origine naturelle ou synthétique.

Parmi les fibres, filaments ou fils 23 d'origine naturelle, on peut distinguer les fibres animales et les fibres végétales. Parmi les fibres animales, on peut citer la soie ou les poils de divers mammifères utilisés pour fabriquer la laine. Parmi les fibres d'origine végétale, on peut citer par exemple le coton, le lin, le chanvre ou la viscose. Des mélanges de ces différentes fibres d'origine animale et/ou végétale peuvent également être adaptés.

Parmi les fibres, filaments ou fils 23 synthétiques ou artificiels, on peut citer par exemple les polyamides, polyesters, polyacryliques, polyéthylènes, chlorofibres, polypropylènes, aramides, verres, polyuréthannes, carbones, polybenzimidazole, polyéther-éthercétone, polysulfure de phénylène, phénolique. L'homme du métier dispose d'une grande variété de matériaux synthétiques utiles dans le domaine textile, dont les applications varient en fonction de l'utilisation envisagée.

Par ailleurs, il est connu de mélanger entre elles des fibres 23 naturelles et synthétiques. Aussi, la base textile 2 peut comprendre un mélange de différents types de fibres, filaments et/ou fils, d'origine naturelle et/ou synthétique.

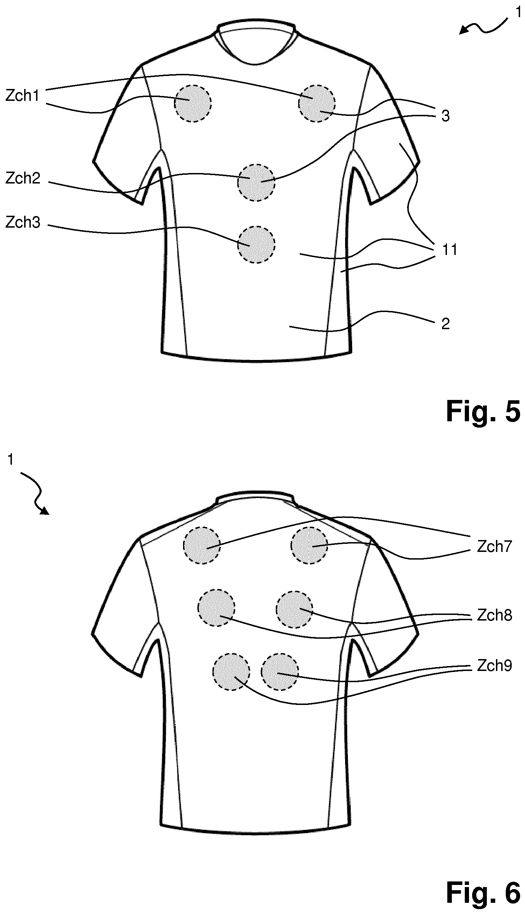

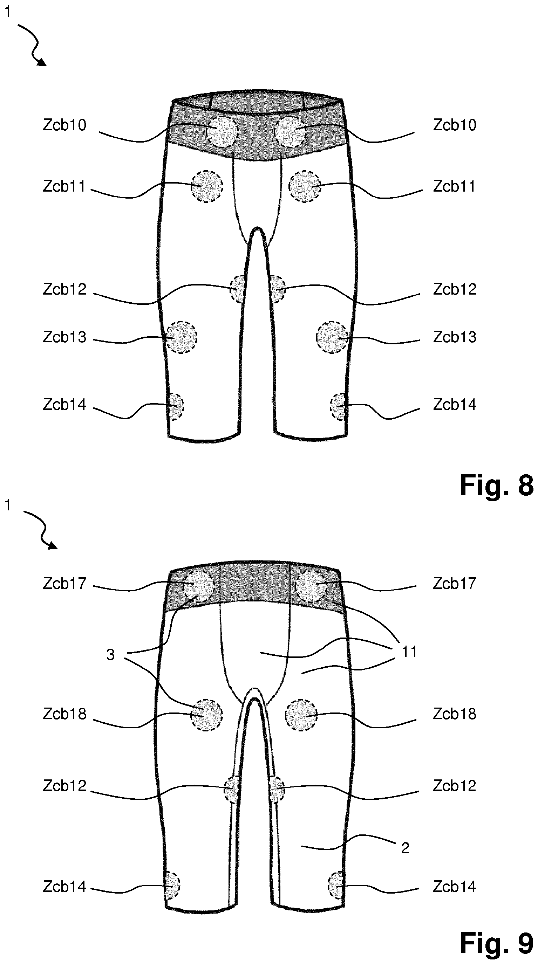

La base textile 2 est le composant principal de plusieurs panneaux 11 assemblés entre eux, selon un patronage déterminé, pour former le vêtement 1, comme on le voit dans les figures 5 à 9. L'assemblage entre les panneaux est réalisé par tout moyen approprié. Ce peut être par couture, collage... Dans une variante, le vêtement 1 est réalisé par un seul panneau de textile selon un procédé spécifique.

Selon l'invention, certaines parties ciblées de la surface interne 21 de la base textile 2 sont recouvertes ou « formées » par un revêtement réflecteur 3 constitué d'au moins un matériau absorbant et restituant des rayonnements infrarouges lointains (IRL). Dans cet exemple, le matériau absorbant et restituant des rayonnements infrarouges lointains (IRL) utilisé est un matériau comprenant au moins un oxyde métallique minéral et notamment, un des oxydes suivants : Al₂O₃, Fe₂O₃, TiO₂, Cr₂O₃, SiO₂. MgO, ZrO₂, MnO₂, Co₂O₃, Y₂O₃ et leurs mélanges.

Pour lier le revêtement réflecteur 3 à la base textile 2, plusieurs techniques peuvent être utilisées.

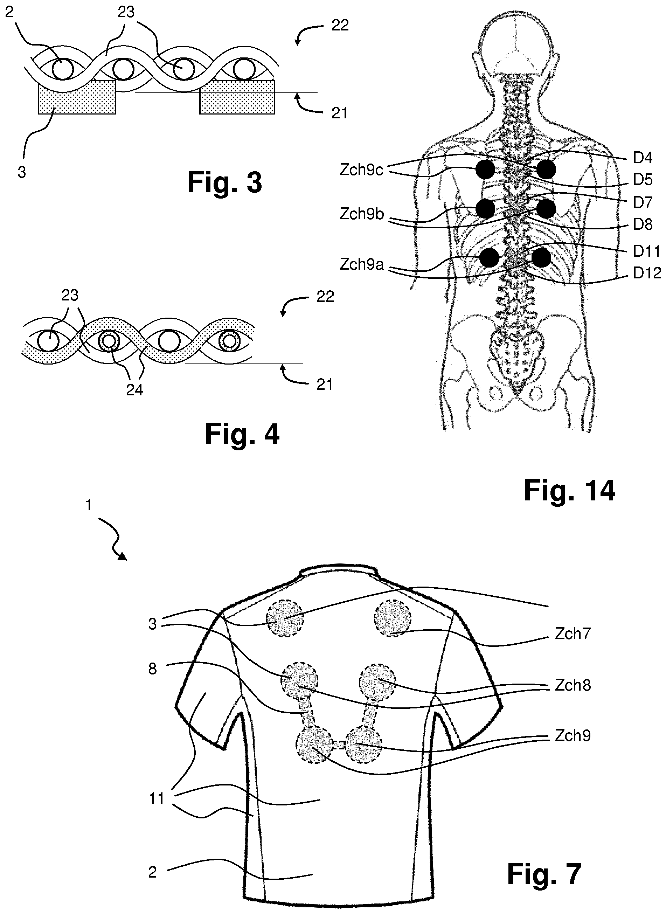

L'association peut être réalisée par impression, collage ou enduction d'un composé comprenant au moins un oxyde métallique sur la base textile. Dans cet exemple, illustré à la figure 3, le revêtement réflecteur 3 recouvre la base textile 2. Selon un exemple, le revêtement réflecteur forme une surcouche dont l'épaisseur est inférieure à un millimètre. Cela permet de peu rigidifier la base textile et d'apporter de la souplesse au vêtement afin de ne pas trop entraver la liberté de mouvement de l'utilisateur.

Alternativement, le revêtement réflecteur peut être réalisé en utilisant des fibres, filaments ou fils 24 incorporant un matériau comprenant au moins un oxyde métallique. Comme illustré à la figure 4, les fibres, filaments ou fils 24 comprennent une âme, constituée d'un matériau sans oxyde métallique, gainé par un enrobage constitué d'un matériau intégrant au moins un oxyde métallique. Selon une variante, les fibres, filaments ou fils 24 sont entièrement constitués d'un matériau intégrant au moins un oxyde métallique. Dans tous ces cas, les fibres, filaments ou fils 24 sont directement incorporés à la base textile par tissage, tricotage ou broderie. Ces fibres, filaments ou fils 24, incorporant au moins un matériau comprenant au moins un oxyde métallique, peuvent être combinés avec des fibres, filaments ou fils 23, de nature différente, sans oxyde métallique. La base textile peut être réalisée avec des procédés permettant l'utilisation des fibres, filaments ou fils 24 incorporant un tel matériau uniquement dans des zones précises. Par exemple, ce peut être un assemblage par « bodymapping ». Ainsi, dans ces modes de réalisation, comme représenté à la figure 4, le revêtement réflecteur 3 forme une partie de la base textile 2.

En variante, le revêtement réflecteur est ménagé sur des pièces distinctes destinées à être assemblées, de manière amovible, à la base textile. Ainsi, chaque pièce distincte ou patch comprend des moyens d'accroche destinés à coopérer avec des moyens d'accroches complémentaires placés sur la surface interne de la base textile au niveau de zones déterminées. Cette coopération entre les moyens d'accroche permet d'assurer le maintien en place et en position du revêtement réflecteur sur le vêtement. De tels moyens d'accroche peuvent être des boutons pression, des crochets/boucles de type VELCRO ^{®}... Cette construction utilisant des patchs rapportés amovibles permet de personnaliser les effets escomptés. Grâce à cette modularité, l'utilisateur, en fonction de son besoin, de son état de forme, de l'activité pratiquée, peut placer ses patchs uniquement dans certaines zones ciblées et ne pas en mettre dans d'autres zones prévues sur le vêtement.

Ainsi, en usage, quelle que soit la solution d'accroche choisie, le revêtement réflecteur est directement solidaire de la surface interne de la base textile. Il n'y a pas de mouvement relatif entre le revêtement réflecteur et la surface interne de la base textile.

Certaines propriétés d'un matériau comprenant au moins un oxyde métallique peuvent s'expliquer par le phénomène physiologique suivant. Le corps émet un rayonnement comprenant notamment des infrarouges. Lorsque ce rayonnement rencontre ledit matériau, celui-ci agit comme un miroir en absorbant une partie des rayonnements infrarouges lointains (IRL) et les renvoyant vers le corps. Ces rayonnements infrarouges lointains (IRL) traversent alors la peau pour stimuler la microcirculation d'organes internes. L'invention consiste à cibler les organes à stimuler en sollicitant le moins possible les organes voisins. Les organes stimulés peuvent être, par exemple, des muscles, des nerfs, l'estomac, l'intestin...

Pour que l'effet de ce type de matériau soit optimum, il est préférable que le revêtement réflecteur 3 soit en contact direct avec la peau. Aussi, selon un mode de réalisation, le vêtement est destiné à être en contact direct avec la peau de l'utilisateur. De tels vêtements sont, par exemple, des tee-shirts, des débardeurs, des shorts, des corsaires, des pantalons, des collants, des chaussettes.

De plus, selon un mode de réalisation, pour maintenir le contact contre la peau et le bon positionnement des zones ciblées Zc du revêtement réflecteur 3, la base textile 2 est un textile compressif ou partiellement compressif, c'est-à-dire, exerçant un légère pression, uniforme ou ponctuelle, sur le corps de l'utilisateur. L'effet compressif peut, par exemple, être obtenu par l'utilisation de fibres, filaments ou fils 23 en élasthanne.

Selon l'invention, le vêtement comprend plusieurs zones ciblées distinctes Zc pour lesquelles la base textile 2 est majoritairement recouverte ou formée du revêtement réflecteur 3. Pour que l'effet bénéfique du revêtement soit efficace et que la proprioception soit optimale, ces zones ciblées doivent se distinguer et, pour cela, doivent être entourées d'une portion de vêtement pour laquelle, la présence du revêtement est moins importante. Ainsi, le rayonnement restitué par le matériau comprenant au moins un oxyde métallique est localisé sur les organes ciblés. Le corps va mieux réagir à ces signaux, sans être perturbé par la présence de ce type de matériau aux alentours de ces zones. Ainsi, cette construction permet de concentrer la présence du revêtement sur au moins deux zones déterminées distinctes.

L'invention définit ces zones ciblées Zc et ces zones périphériques Zp. Ainsi, chaque zone ciblée Zc est définie par un disque 4, de diamètre 41 au moins égal à 1,5 centimètre, à l'intérieur duquel la base textile 2 est recouverte ou formée du revêtement réflecteur 3 sur 90% de la surface interne S4 du disque 4. Par ailleurs, chaque zone ciblée Zc est entourée d'une zone périphérique Zp formant un anneau 5 délimité par un diamètre interne 51 égal au diamètre 41 dudit disque 4 et par un diamètre externe 521 d'un cercle 52 égal à quatorze centimètres. Dans cette zone périphérique Zp, la base textile 2 est recouverte ou formée du revêtement réflecteur 3 sur moins de 50% de la surface interne S5 de l'anneau 5. De plus, la totalité des revêtements réflecteurs 3 associés à la surface interne 21 de la base textile 2 recouvre ou forme entre 5% et 50% de la totalité de la surface interne 21 de la base textile 2.

Pour que le revêtement produise un effet bénéfique, il faut que chaque zone ciblée s'inscrive dans un disque 4 ayant un diamètre 41 supérieur à 1,5 centimètre. En dessous, la stimulation n'est pas suffisante pour avoir un bénéfice significatif.

Selon une variante, le diamètre 41 du disque 4 définissant plusieurs zones ciblées Zc est inférieur à huit centimètres. Cette limite supérieure permet de mieux concentrer l'information reçue par le corps et améliore la réaction du corps à cette sollicitation produite par ce type de matériau. Au-delà, la diffusion interne du rayonnement IRL est moins efficace.

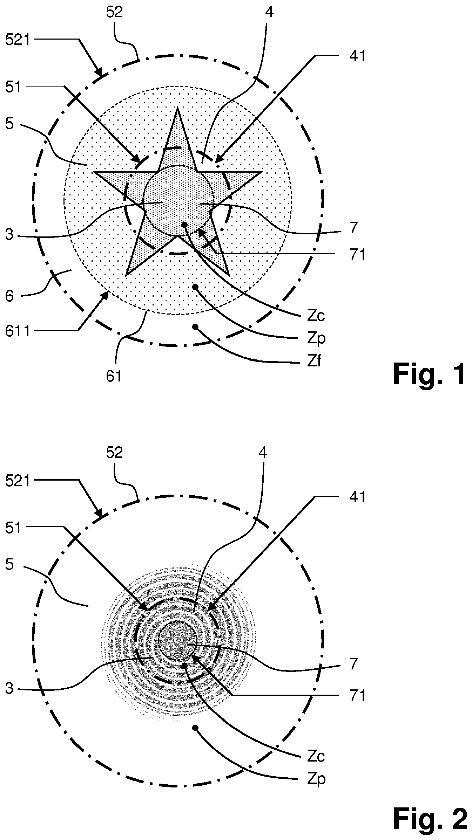

Dans la zone périphérique Zp, le revêtement réflecteur 3 recouvre ou forme entre 0 et 50% de la base textile. Autrement dit, dans la zone périphérique Zp, la surface interne de la base textile peut, soit être recouverte ou formée par revêtement réflecteur dans une proportion bien moindre, inférieure à 50%, que dans la zone ciblée, soit cette zone est exempt de revêtement. Ainsi, selon un mode de réalisation, dans cette zone périphérique, aucun revêtement réflecteur 3 ne recouvre la base textile 2. Selon une autre variante, la zone périphérique Zp de plusieurs zones ciblées Zc comprend une zone libre Zf formant un anneau 6 délimité par un diamètre interne 611 d'un cercle 61, le diamètre interne 611 étant égal à onze centimètres et par le diamètre externe 521 du cercle 52, le diamètre externe 521 étant égal à quatorze centimètres. Dans cette zone libre Zf, aucun revêtement réflecteur 3 ne couvre ou ne forme la base textile 2. Dans ce cas, comme on le voit sur les figures 1 et 2, on obtient trois secteurs autour de certaines zones ciblées Zc. Un premier secteur, dont la base textile est recouverte ou formée du revêtement réflecteur sur 90% de la surface interne, couvre un disque 4, de diamètre 41 au moins égal à 1,5 centimètre. Un deuxième secteur intermédiaire, dont la base textile est recouverte ou formée du revêtement réflecteur sur une moindre proportion, moins de 50%, de la surface interne, couvre un anneau délimité par un diamètre interne 51 égal au diamètre 41 dudit disque 4 et par un diamètre externe 611 d'un cercle 61 égal à onze centimètres. Un troisième secteur, pour laquelle aucun revêtement réflecteur 3 ne couvre ou ne forme la base textile 2, couvre un anneau 6 délimité par un diamètre interne 611 d'un cercle 61, le diamètre interne 611 étant égal à onze centimètres et par le diamètre externe 521 du cercle 52, le diamètre externe 521 étant égal à quatorze centimètres. Cela permet d'obtenir une concentration de revêtement réflecteur dégressive plus on s'écarte de la zone ciblée Zc concernée. L'absence de revêtement réflecteur 3 dans cette zone libre Zf renforce davantage les effets et la proprioception résultant de la présence du revêtement réflecteur 3 dans la zone ciblée correspondante.

Le revêtement réflecteur 3 dans une zone ciblée Zc peut prendre toutes formes à partir du moment où elle peut être définie selon les critères décrits précédemment. Ainsi, chaque zone ciblée, dont la base textile est recouverte ou formée du revêtement réflecteur sur 90% de la surface interne de cette zone, doit pouvoir être définie par un disque comme nous l'avons caractérisé auparavant et être cernée par une zone périphérique ayant une concentration de revêtement réflecteur moindre. Le revêtement réflecteur 3 peut donc reproduire des formes géométriques simples telles qu'un carré, un losange, un disque ou encore une étoile, comme représenté à la figure 1. Alternativement, il peut reproduire un motif géométrique plus complexe, comme par exemple celui représenté à la figure 2. La couche de revêtement céramique 3 peut recouvrir entièrement la base textile 2 sur au moins une partie dense Zd de la zone ciblée. Cette zone dense Zd est définie par un disque 7 de diamètre 71. Dans ce cas, on aura un recouvrement du revêtement réflecteur de 100% de la surface interne S7 du disque 7 de diamètre 71. Le diamètre 71 du disque 7 est au moins égal à 1,5 centimètre.

Selon un mode de réalisation, le revêtement réflecteur 3 forme une spirale ou une combinaison de spirales, comme illustré à la figure 2, centrée sur la zone ciblée. Dans cet exemple, le centre de la spirale ou de la combinaison de spirales forme un disque 7 de diamètre 71 supérieur à 1,5 centimètre. Le revêtement réflecteur 3 remplit alors la totalité de la surface interne S7 du disque 7. Dans cet exemple, chaque spirale est définie par une courbe formant une bande de revêtement réflecteur 3 de largeur au moins égale à deux millimètres. Avantageusement, l'espace sans revêtement réflecteur 3 entre deux courbes de revêtement réflecteur 3, mesuré selon une direction radiale, augmente plus on s'écarte du centre de la spirale. Dans cet exemple, la bande de revêtement réflecteur 3 formant une courbe d'une spirale est divisée en deux par une bande médiane sans couche de revêtement réflecteur 3 à partir d'une certaine distance du centre de la spirale. La largeur de cette bande médiane sans couche de revêtement réflecteur 3 croît, plus on s'écarte du centre de la spirale.

Selon un mode de réalisation, représenté à la figure 7, plusieurs zones ciblées Zc sont reliées entre elles par des bandes 8 composées de revêtement réflecteur 3 dans la mesure où la zone périphérique Zp des zones ciblées Zc respecte toujours la condition de concentration de matériau comprenant au moins un oxyde métallique.

Pour renforcer l'efficacité de l'effet du revêtement réflecteur, il est préférable d'isoler les zones ciblées afin de mieux concentrer les rayonnements infrarouges lointains sur un point déterminé. La zone périphérique Zp assure un premier niveau d'isolement. Selon un mode de réalisation, pour renforcer cet isolement, la majeure partie du vêtement comprend une base textile sans couche de revêtement réflecteur 3, entre les zones ciblées Zc. Par exemple, plus de 50% de la surface interne 21 de la base textile 2 ne comprend pas de couche de revêtement réflecteur 3.

Grâce à l'invention, en enfilant ce type de vêtement, l'utilisateur est assuré de placer correctement des portions de recouvrement réflecteur au niveau de zones précises, en vis-à-vis de parties de corps où l'on souhaite une interaction ciblée entre le matériau composé d'au moins un oxyde métallique et le corps. Ces emplacements spécifiques reproduisent une cartographie correspondant à des points particuliers à solliciter en fonction d'une activité déterminée. Ainsi, en fonction de l'activité pratiquée, l'utilisateur va porter un vêtement spécifique, dédié à cette activité.

Selon un mode de réalisation, le vêtement est conçu pour qu'au moins une zone ciblée Zc munie du revêtement réflecteur 3 soit positionnée sur le vêtement de sorte qu'elle recouvre sensiblement, lorsque le vêtement 1 est porté, un des emplacements spécifiques du corps suivant :
- selon l'invention, une zone ciblée haute Zch1 localisée en vis-à-vis de la partie haute externe du muscle du grand pectoral, sous la clavicule ;
- selon l'invention, une zone ciblée haute Zch2 localisée en vis-à-vis du processus ou appendice xiphoïde du sternum ;
- selon l'invention, une zone ciblée haute Zch3 localisée en vis-à-vis de l'estomac à mi-distance entre l'appendice xiphoïde et le nombril,
- selon un exemple ne formant pas partie de l'invention, une zone ciblée haute Zch4 localisée en vis-à-vis du V deltoïdien, au niveau de l'attache des muscles deltoïdes ;
- selon un exemple ne formant pas partie de l'invention, une zone ciblée haute Zch5 localisée en vis-à-vis de la partie haute des muscles épicondyliens, au niveau des attaches hautes du muscle brachio-radial, du muscle long extenseur radial du carpe, du muscle court extenseur radial du carpe et du muscle extenseur des doigts ;
- selon un exemple ne formant pas partie de l'invention, une zone ciblée haute Zch6 localisée en vis-à-vis du ligament annulaire du Carpe au niveau de la face antérieure du poignet ;
- selon l'invention, une zone ciblée haute Zch7 localisée en vis-à-vis d'une zone bordée par la partie du muscle trapèze supérieur, par le muscle supra-épineux et par le muscle élévateur de la scapula ;
- selon un exemple ne formant pas partie de l'invention, une zone ciblée haute Zch8 localisée en vis-à-vis d'une zone en arrière de l'épaule, contre l'omoplate ou scapula sur le muscle grand rond ;
- selon l'invention, une zone ciblée haute Zch9b localisée en vis-à-vis de deux zones dorsale situées de part et d'autre des vertèbres dorsales ou thoraciques D7, D8 ;
- selon l'invention, une zone ciblée haute Zch9c localisée en vis-à-vis de deux zones dorsale situées de part et d'autre des vertèbres dorsales ou thoraciques D4, D5 ;
- selon l'invention, une zone ciblée haute Zch9a localisée en vis-à-vis de deux zones dorsale situées de part et d'autre des vertèbres dorsales ou thoraciques D11, D12 ;
- selon un exemple ne formant pas partie de l'invention, une zone ciblée basse Zcb10 localisée sur les hypochondres en vis-à-vis des intestins (caecum et sigmoïde) ;
- selon un exemple ne formant pas partie de l'invention, une zone ciblée basse Zcb11 localisée en vis-à-vis des attaches hautes des muscles du quadriceps ;
- selon un exemple ne formant pas partie de l'invention, une zone ciblée basse Zcb12 localisée en vis-à-vis de la partie médiane du muscle sartorius ou couturier, à l'intérieur de la cuisse ;
- selon un exemple ne formant pas partie de l'invention, une zone ciblée basse Zcb13 localisée en vis-à-vis de la partie inférieure du muscle vaste latéral ;
- selon un exemple ne formant pas partie de l'invention, une zone ciblée basse Zcb14 localisée en vis-à-vis de la partie haute du muscle tibial antérieur ;
- selon un exemple ne formant pas partie de l'invention, une zone ciblée basse Zcb15 localisée en vis-à-vis des parties médianes du muscle soléaire, du muscle long extenseur des orteils, et du muscle long fibulaire [péronier] et de la partie haute du muscle court fibulaire [péronier], cette zone étant située au tiers inférieur de la partie latérale de la jambe ;
- selon un exemple ne formant pas partie de l'invention, une zone ciblée basse Zcb16 localisée en vis-à-vis des parties médianes du muscle tibial postérieur et du muscle long fléchisseur des orteils, cette zone étant située au tiers inférieur et interne de la partie médiale de la jambe ;
- selon un exemple ne formant pas partie de l'invention, une zone ciblée basse Zcb17 localisée en vis-à-vis de la partie haute du muscle grand fessier et de la partie postérieure haute du muscle moyen fessier ;
- selon un exemple ne formant pas partie de l'invention, une zone ciblée basse Zcb18 localisée en vis-à-vis des attaches hautes des muscles des ischio-jambiers ;
- selon un exemple ne formant pas partie de l'invention, une zone ciblée basse Zcb19 localisée en vis-à-vis de l'insertion des muscles gastrocnémiens sur le tendon calcanéen ;
- selon un exemple ne formant pas partie de l'invention, une zone ciblée basse Zcb20 localisée en vis-à-vis d'une zone positionnée en arrière de la malléole médiale ou interne et en avant du tendon calcanéen (tendon d'Achille) ;
- selon un exemple ne formant pas partie de l'invention, une zone ciblée basse Zcb21 localisée en vis-à-vis d'une zone positionnée sous la malléole latérale ou externe et légèrement en avant, sur la capsule articulaire de la cheville ;
- selon un exemple ne formant pas partie de l'invention, une zone ciblée basse Zcb22 localisée face dorsale du pied, en vis-à-vis d'une zone positionnée en haut de l'espace intermétatarsien entre le 4e et 5e espace métatarsien ;
- selon un exemple ne formant pas partie de l'invention, une zone ciblée basse Zcb23 localisée, sous le pied, en vis-à-vis d'une zone positionnée en arrière des métatarses sur la semelle de Lejars ;
- selon un exemple ne formant pas partie de l'invention, une zone ciblée basse Zcb24 localisée, sous le pied, en vis-à-vis d'une zone positionnée sous le premier métatarse.

La zone ciblée haute Zch1 permet de détendre les pectoraux et de libérer les côtes supérieures, ce qui permet de faciliter la respiration.

La zone ciblée haute Zch2 permet de détendre le plexus solaire/cardiaque et de réguler les systèmes nerveux sympathique et parasympathique. Les muscles du diaphragme sont mieux détendus ce qui aide également la respiration.

La zone ciblée haute Zch3 permet de soulager les troubles éventuels de l'estomac.

La zone ciblée haute Zch4 permet de détendre l'ensemble des deltoïdes pour un meilleur relâchement des épaules.

La zone ciblée haute Zch5 permet d'éviter l'épicondylite ou une inflammation des tendons au niveau du coude.

La zone ciblée haute Zch6 permet une meilleure irrigation des vaisseaux de la main ce qui va accroître les sensations au niveau des doigts, notamment lorsqu'il fait froid. Elle permet également d'avoir une action de régulation du système para et ortho sympathique.

La zone ciblée haute Zch7 permet de libérer de la rotation de la nuque, des vertèbres cervicales.

La zone ciblée haute Zch8 permet l'ouverture et le contrôle positionnel de l'épaule.

Les zones ciblées hautes Zch9a, Zch9b et Zch9c permettent de faciliter la rotation du torse autour de la colonne vertébrale. De plus, cela agit également sur les muscles du diaphragme de sorte à faciliter la respiration. La zone ciblée haute Zch9b étant localisée au même emplacement que la partie dorsale de la bande/aile d'une brassière sportive, cette zone ciblée haute peut être décalée au niveau de la zone ciblée haute Zch9c pour les vêtements féminins.

La zone ciblée haute Zcb10 permet de soulager les tensions intestinales notamment les crispations au niveau des muscles lisses du péristaltisme intestinal.

Les zones ciblées basses Zcb11, Zcb12, Zcb13 et Zcb18 agissent sur les principaux muscles des cuisses. Elles améliorent leur irrigation ce qui va retarder la fatigue musculaire au niveau des cuisses, lors d'effort, et aide à la récupération.

La zone ciblée basse Zcb14 permet la stimulation du muscle tibial antérieur garant du bon maintien dynamique de la cheville.

Les zones ciblées basses Zcb15 et Zcb16 permettent d'assurer un bon maintien dynamique du pied.

La zone ciblée basse Zcb17 permet de soulager le muscle moyen fessier.

La zone ciblée basse Zcb19 permet d'éviter d'avoir des crampes au mollet.

La zone ciblée basse Zcb20 permet d'augmenter la circulation sanguine vers le dessous du pied, procurant ainsi une aide à la stabilisation de cheville. Elle permet aussi de détendre le tendon d'Achille.

La zone ciblée basse Zcb21 contribue à la stabilisation de la cheville.

La zone ciblée basse Zcb22 permet de travailler entre autre sur l'os cuboïde du pied et améliorer la stabilisation du bassin.

La zone ciblée basse Zcb23 permet de détendre des muscles plantaires.

La zone ciblée basse Zcb24 permet de détendre et de stabiliser le premier métatarse, lors de l'appui à la course.

Pour la pratique de la course à pied, de la randonnée, il est préférable de stimuler au moins une ou plusieurs des zones ciblées suivantes : ZCh1, ZCh2, ZCh3, ZCh7, ZCh9a, ZCh9b, ZCh9c, ZCb10, ZCb11, ZCb12, ZCb13, ZCb14, ZCb17, ZCb18, Zcb19, Zcb20, Zcb21, Zcb22, Zcb24.

Pour la pratique du ski de fond, il est préférable de stimuler au moins une ou plusieurs des zones ciblées suivantes : ZCh1, ZCh2, ZCh3, ZCh4, ZCh5, ZCh6, ZCh7, ZCh9a, ZCh9b, ZCh9c, ZCb10, ZCb11, ZCb12, ZCb13, ZCb14, ZCb15, ZCb16, ZCb17, ZCb18, ZCb19, Zcb20, Zcb21, Zcb22, Zcb23.

Pour la pratique du ski alpin, il est préférable de stimuler au moins une ou plusieurs des zones ciblées suivantes : ZCh1, ZCh2, ZCh3, ZCh7, ZCh9a, ZCh9b, ZCh9c, ZCb10, ZCb11, ZCb12, ZCb13, ZCb14, ZCb15, ZCb16, ZCb17, ZCb18, ZCb19, Zcb20, Zcb23, Zcb24.

Pour la pratique du cyclisme, il est préférable de stimuler au moins une ou plusieurs des zones ciblées suivantes : ZCh1, ZCh2, ZCh3, ZCh5, ZCh6, ZCh7, ZCh9a, ZCh9b, ZCh9c, ZCb10, ZCb11, ZCb12, ZCb13, ZCb14, ZCb17, ZCb18, ZCb19, Zcb20, Zcb23, Zcb24.

Pour la pratique du tennis, il est préférable de stimuler au moins une ou plusieurs des zones ciblées suivantes : ZCh1, ZCh2, ZCh3, ZCh4, ZCh5, ZCh6, ZCh7, ZCh8, ZCh9a, ZCh9b, ZCh9c, ZCb11, ZCb12, ZCb13, ZCb14, ZCb17, ZCb18, ZCb19, Zcb20, Zcb21, Zcb22, Zcb24.

Pour la pratique du basket, il est préférable de stimuler au moins une ou plusieurs des zones ciblées suivantes : ZCh1, ZCh2, ZCh3, ZCh4, ZCh5, ZCh6, ZCh7, ZCh8, ZCh9a, ZCh9b, ZCh9c, ZCb11, ZCb12, ZCb13, ZCb14, ZCb17, ZCb18, ZCb19, Zcb20, Zcb21, Zcb22, Zcb23, Zcb24.

Pour la pratique du baseball, il est préférable de stimuler au moins une ou plusieurs des zones ciblées suivantes : ZCh1, ZCh2, ZCh3, ZCh4, ZCh5, ZCh6, ZCh7, ZCh8, ZCh9a, ZCh9b, ZCh9c, ZCb11, ZCb12, ZCb13, ZCb14, ZCb17, ZCb18, ZCb19, Zcb21, Zcb22, Zcb24. L'invention s'étend à tous les modes de réalisation couverts par les revendications annexées.

### Nomenclature

1- Vêtement
   11- Panneau
2- Base textile
   21- Surface interne
   22- Surface externe
3- Couche de revêtement céramique
4- Disque (Zone ciblée Zc)
   41- Diamètre
5- Anneau (Zone périphérique Zp)
   51- Diamètre interne
   52- Cercle
      521- Diamètre externe
6- Anneau (Zone libre Zf)
   61- Cercle
   611- Diamètre interne
7- Disque (Zone dense Zd)
   71- Diamètre

## Revendications

1. Vêtement (1) comprenant :
- une base textile (2) destinée à recouvrir une partie du corps de l'utilisateur, la base textile comportant une surface interne (21) destinée à être positionnée en vis-à-vis du corps de l'utilisateur et une surface externe (22), opposée à la surface interne et,
- un revêtement réflecteur (3) constitué d'un matériau comprenant au moins un oxyde métallique, le revêtement réflecteur recouvrant ou formant une partie de la surface interne de la base textile, le revêtement réflecteur étant destiné à être en contact direct avec la peau de l'utilisateur,
et le vêtement comprend plusieurs zones ciblées distinctes (Zc), chaque zone ciblée étant définie par un disque (4), de diamètre (41) au moins égal à 1,5 centimètre, à l'intérieur duquel la base textile est recouverte ou formée du revêtement réflecteur sur 90% de la surface interne du disque, chaque zone ciblée étant entourée d'une zone périphérique (Zp) formant un anneau (5) délimité par un diamètre interne (51) égal au diamètre externe dudit disque et par un diamètre externe (521) d'un cercle (52) égal à quatorze centimètres, dans la zone périphérique, la base textile étant recouverte ou formée du revêtement réflecteur entre 0 et 50% de la surface interne de l'anneau,
en ce que
la totalité des revêtements réflecteurs associés à la surface interne de la base textile recouvre ou forme entre 5% et 50% de la totalité de la surface interne de la base textile;
et **caractérisé en ce que** au moins une zone ciblée (Zc) munie du revêtement réflecteur (3) est positionnée sur le vêtement de sorte qu'elle recouvre sensiblement, lorsque le vêtement (1) est porté, un des emplacements spécifiques du corps suivant :
- une zone ciblée haute (Zch1) localisée en vis-à-vis de la partie haute externe du muscle du grand pectoral, sous la clavicule ;
- une zone ciblée haute (Zch2) localisée en vis-à-vis du processus ou appendice xiphoïde du sternum ;
- une zone ciblée haute (Zch3) localisée en vis-à-vis de l'estomac à mi-distance entre l'appendice xiphoïde et le nombril ;
- une zone ciblée haute (Zch7) localisée en vis-à-vis d'une zone bordée par la partie du muscle trapèze supérieur, par le muscle supra-épineux et par le muscle élévateur de la scapula ;
- une zone ciblée haute (Zch9a) localisée en vis-à-vis de deux zones dorsales situées de part et d'autre des vertèbres dorsales ou thoraciques D11, D12 ;
- une zone ciblée haute (Zch9b) localisée en vis-à-vis de deux zones dorsales situées de part et d'autre des vertèbres dorsales ou thoraciques D7, D8 ;
- une zone ciblée haute (Zch9c) localisée en vis-à-vis de deux zones dorsales situées de part et d'autre des vertèbres dorsales ou thoraciques D4, D5 ;

2. Vêtement (1) selon la revendication 1, **caractérisé en ce que** le diamètre externe du disque définissant plusieurs zones ciblées est inférieur à huit centimètres.

3. Vêtement (1) selon l'une des revendications précédentes, **caractérisé en ce que**, dans la zone périphérique, un revêtement réflecteur (3) est présent et recouvre ou forme une partie de la surface interne de la base textile, au niveau de la surface interne, dans une moindre concentration par rapport à celle de la zone ciblée (Zc).

4. Vêtement (1) selon l'une des revendications précédentes, **caractérisé en ce que** la zone périphérique de plusieurs zones ciblées comprend une zone libre (Zf) formant un anneau délimité par un cercle (611) de diamètre égal à onze centimètres et par le cercle (521) de diamètre égal à quatorze centimètres, dans la zone libre, aucun revêtement réflecteur ne couvre ou ne forme une partie de la surface interne de la base textile.

5. Vêtement (1) selon la revendication précédente, **caractérisé en ce que** la zone périphérique comprend un secteur intermédiaire couvrant un anneau délimité par un diamètre interne (51) égal au diamètre (41) dudit disque (4) et par un diamètre externe (611) d'un cercle (61) égal à onze centimètres et dans lequel un revêtement réflecteur (3) est présent et recouvre ou forme une partie de la surface interne de la base textile, au niveau de la surface interne, dans une proportion inférieure à 50% de la surface interne de l'anneau formant le secteur intermédiaire.

6. Vêtement (1) selon l'une des revendications précédentes, **caractérisé en ce que** la couche de revêtement réflecteur (3) recouvre entièrement la base textile (2) sur au moins une partie dense (Zd) de la zone ciblée (Zc), cette partie dense étant définie par un disque (7) dont le diamètre (71) est au moins égal à 1,5 centimètre.

7. Vêtement (1) selon l'une des revendications précédentes, **caractérisé en ce que** plusieurs zones ciblées sont reliées par des zones de liaison munies de revêtement réflecteur.

8. Vêtement (1) selon l'une des revendications précédentes, **caractérisé en ce que** plusieurs revêtements réflecteurs sont associés avec la base textile par impression, collage, enduction, tissage, tricotage ou broderie.

9. Vêtement (1) selon l'une des revendications précédentes, **caractérisé en ce que** le revêtement réflecteur est ménagé sur des pièces distinctes destinées à être assemblées de manière amovible sur la base textile.

10. Vêtement (1) selon l'une des revendications précédentes, **caractérisé en ce que en ce que** la base textile est un textile tricoté, tissé ou non-tissé.

11. Vêtement (1) selon l'une des revendications précédentes, **caractérisé en ce que** le revêtement réflecteur (3) forme une spirale ou une combinaison de spirales.

12. Vêtement (1) selon l'une des revendications précédentes, **caractérisé en ce que** le revêtement réflecteur (3) forme une surcouche dont l'épaisseur est inférieure à un millimètre.

13. Vêtement (1) selon l'une des revendications précédentes, **caractérisé en ce que** le revêtement réflecteur (3) comprend au moins un des oxydes métalliques minéraux suivants : Al₂O₃, Fe₂O₃, TiO₂, Cr₂O₃, SiO₂. MgO, ZrO₂, MnO₂, Co₂O₃, Y₂O₃ et leurs mélanges.

## Patentansprüche

1. Kleidungsstück (1), umfassend:
- eine textile Basis (2), die dazu bestimmt ist, einen Teil des Körpers des Benutzers zu bedecken, wobei die textile Basis eine Innenfläche (21), die dazu bestimmt ist, gegenüber dem Körper des Benutzers positioniert zu werden, und eine Außenfläche (22), die zur Innenfläche entgegengesetzt ist, aufweist, und
- eine reflektierende Beschichtung (3), die aus einem Material besteht, das mindestens ein Metalloxid umfasst, wobei die reflektierende Beschichtung einen Teil der Innenfläche der textilen Basis bedeckt oder bildet, wobei die reflektierende Beschichtung dazu bestimmt ist, in direktem Kontakt mit der Haut des Benutzers zu sein,
und das Kleidungsstück mehrere verschiedene gezielte Bereiche (Zc) umfasst, wobei jeder gezielte Bereich durch eine Scheibe (4) mit einem Durchmesser (41) von mindestens 1,5 Zentimeter definiert ist, in deren Inneren die textile Basis auf 90 % der Innenfläche der Scheibe von der reflektierenden Beschichtung bedeckt oder gebildet wird, wobei jeder gezielte Bereich von einem Randbereich (Zp) umgeben ist, der einen Ring (5) bildet, der durch einen Innendurchmesser (51) gleich dem Außendurchmesser der Scheibe und durch einen Außendurchmesser (521) eines Kreises (52) von vierzehn Zentimetern begrenzt wird, wobei in dem Randbereich die textile Basis zwischen 0 und 50 % der Innenfläche des Rings von der reflektierenden Beschichtung bedeckt oder gebildet wird,
dadurch, dass die Gesamtheit der reflektierenden Beschichtungen, die mit der Innenfläche der textilen Basis verbunden sind, zwischen 5 % und 50 % der Gesamtheit der Innenfläche der textilen Basis bedeckt oder bildet;
und **dadurch gekennzeichnet, dass** mindestens ein gezielter Bereich (Zc), der mit der reflektierenden Beschichtung (3) versehen ist, auf dem Kleidungsstück so positioniert ist, dass er im Wesentlichen, wenn das Kleidungsstück (1) getragen wird, eine der folgenden spezifischen Stellen des Körpers bedeckt:
- einen oberen gezielten Bereich (Zch1), der gegenüber dem äußeren oberen Teil des großen Brustmuskels liegt, unter dem Schlüsselbein;
- einen oberen gezielten Bereich (Zch2), der gegenüber dem Schwertfortsatz des Brustbeins liegt;
- einen oberen gezielten Bereich (Zch3), der gegenüber dem Magen in der Mitte zwischen dem Schwertfortsatz und dem Nabel liegt;
- einen oberen gezielten Bereich (Zch7), der gegenüber einem Bereich liegt, der vom Teil des oberen Trapezmuskels, vom Obergrätenmuskel und vom Schulterblattheber gesäumt wird;
- einen oberen gezielten Bereich (Zch9a), der gegenüber von zwei Rückenbereichen liegt, die beidseits der Rücken- oder Brustwirbel D11, D12 gelegen sind;
- einen oberen gezielten Bereich (Zch9b), der gegenüber von zwei Rückenbereichen liegt, die beidseits der Rücken- oder Brustwirbel D7, D8 gelegen sind;
- einen oberen gezielten Bereich (Zch9c), der gegenüber von zwei Rückenbereichen liegt, die beidseits der Rücken- oder Brustwirbel D4, D5 gelegen sind.

2. Kleidungsstück (1) nach Anspruch 1, **dadurch gekennzeichnet, dass** der Außendurchmesser der Scheibe, die mehrere gezielte Bereiche definiert, weniger als acht Zentimeter beträgt.

3. Kleidungsstück (1) nach einem der vorhergehenden Ansprüche, **dadurch gekennzeichnet, dass** in dem Randbereich eine reflektierende Beschichtung (3) vorhanden ist und einen Teil der Innenfläche der textilen Basis, an der Innenfläche, in einer geringeren Konzentration in Bezug auf die des gezielten Bereichs (Zc) bedeckt oder bildet.

4. Kleidungsstück (1) nach einem der vorhergehenden Ansprüche, **dadurch gekennzeichnet, dass** der Randbereich von mehreren gezielten Bereichen einen freien Bereich (Zf) umfasst, der einen Ring bildet, der durch einen Kreis (611) mit einem Durchmesser von elf Zentimetern und durch den Kreis (521) mit einem Durchmesser von vierzehn Zentimetern begrenzt wird, wobei in dem freien Bereich keine reflektierende Beschichtung einen Teil der Innenfläche der textilen Basis bedeckt oder bildet.

5. Kleidungsstück (1) nach dem vorhergehenden Anspruch, **dadurch gekennzeichnet, dass** der Randbereich einen Zwischensektor umfasst, der einen Ring bedeckt, der durch einen Innendurchmesser (51) gleich dem Durchmesser (41) der Scheibe (4) und durch einen Außendurchmesser (611) eines Kreises (61) von elf Zentimetern begrenzt wird und in dem eine reflektierende Beschichtung (3) vorhanden ist und einen Teil der Innenfläche der textilen Basis, an der Innenfläche, in einem Verhältnis von weniger als 50 % der Innenfläche des den Zwischensektor bildenden Rings bedeckt oder bildet.

6. Kleidungsstück (1) nach einem der vorhergehenden Ansprüche, **dadurch gekennzeichnet, dass** die reflektierende Beschichtungsschicht (3) die textile Basis (2) auf mindestens einem dichten Teil (Zd) des gezielten Bereichs (Zc) vollständig bedeckt, wobei dieser dichte Teil durch eine Scheibe (7) definiert ist, deren Durchmesser (71) mindestens 1,5 Zentimeter beträgt.

7. Kleidungsstück (1) nach einem der vorhergehenden Ansprüche, **dadurch gekennzeichnet, dass** mehrere gezielte Bereiche durch Verbindungsbereiche verbunden sind, die mit einer reflektierenden Beschichtung versehen sind.

8. Kleidungsstück (1) nach einem der vorhergehenden Ansprüche, **dadurch gekennzeichnet, dass** mehrere reflektierende Beschichtungen mit der textilen Basis durch Drucken, Kleben, Beschichten, Weben, Stricken oder Sticken verbunden sind.

9. Kleidungsstück (1) nach einem der vorhergehenden Ansprüche, **dadurch gekennzeichnet, dass** die reflektierende Beschichtung auf verschiedenen Teilen ausgebildet ist, die dazu bestimmt sind, lösbar an der textilen Basis angebracht zu werden.

10. Kleidungsstück (1) nach einem der vorhergehenden Ansprüche, **dadurch gekennzeichnet, dass** die textile Basis ein gestricktes, gewebtes oder nicht gewebtes Textil ist.

11. Kleidungsstück (1) nach einem der vorhergehenden Ansprüche, **dadurch gekennzeichnet, dass** die reflektierende Beschichtung (3) eine Spirale oder eine Kombination aus Spiralen bildet.

12. Kleidungsstück (1) nach einem der vorhergehenden Ansprüche, **dadurch gekennzeichnet, dass** die reflektierende Beschichtung (3) eine Deckschicht bildet, deren Dicke weniger als einen Millimeter beträgt.

13. Kleidungsstück (1) nach einem der vorhergehenden Ansprüche, **dadurch gekennzeichnet, dass** die reflektierende Beschichtung (3) mindestens eines der folgenden mineralischen Metalloxide umfasst: Al₂O₃, Fe₂O₃, TiO₂, Cr₂O₃, SiO₂, MgO, ZrO₂, MnO₂, Co₂O₃, Y₂O₃ und ihre Mischungen.

## Claims

1. Garment (1) comprising:
- a textile base (2) intended to cover a part of the body of the user, the textile base having an internal surface (21) intended to be positioned facing the body of the user and an external surface (22), which is opposite the internal surface and,
- a reflective coating (3) constituted of a material comprising at least one metal oxide, the reflective coating covering or forming a part of the internal surface of the textile base, the reflective coating being intended to be in direct contact with the skin of the user,
and
the garment comprising a plurality of distinct targeted zones (Zc), each targeted zone being defined by a disc (4), of diameter (41) at least equal to 1.5 centimetres, inside which the textile base is covered or formed by the reflective coating over 90% of the internal surface of the disc, each targeted zone being surrounded by peripheral zone (Zp) forming a ring (5) delimited by an internal diameter (51) equal to the external diameter of said disc and by an external diameter (521) of a circle (52) equal to fourteen centimetres, in the peripheral zone, the textile base being covered or formed by the reflective coating over between 0 and 50% of the internal surface of the ring,
**characterized in that**
the totality of the reflective coatings associated with the internal surface of the textile base covers or forms between 5% and 50% of the totality of the internal surface of the textile base;
and **characterized in that**
at least one targeted zone (Zc) provided with the reflective coating (3) is positioned on the garment such that it substantially covers, when the garment (1) is worn, one of the following specific locations on the body:
- a high targeted zone (Zch1) located facing the external high part of the pectoralis major muscle, beneath the clavicle;
- a high targeted zone (Zch2) located facing the xiphoid process or appendix of the sternum;
- a high targeted zone (Zch3) located facing the stomach midway between the xiphoid appendix and the navel;
- a high targeted zone (Zch7) located facing a zone bordered by the part of the upper trapezius muscle, by the supraspinatus muscle and by the levator scapulae muscle;
- a high targeted zone (Zch9a) located facing two dorsal zones situated on either side of the dorsal or thoracic vertebrae D11, D12;
- a high targeted zone (Zch9b) located facing two dorsal zones situated on either side of the dorsal or thoracic vertebrae D7, D8;
- a high targeted zone (Zch9c) located facing two dorsal zones situated on either side of the dorsal or thoracic vertebrae D4, D5.

2. Garment (1) according to Claim 1, **characterized in that** the external diameter of the disc defining a plurality of targeted zones is less than eight centimetres.

3. Garment (1) according to either of the preceding claims, **characterized in that**, in the peripheral zone, a reflective coating (3) is present and covers or forms a part of the internal surface of the textile base, at the internal surface, in a lower concentration relative to that of the targeted zone (Zc).

4. Garment (1) according to one of the preceding claims, **characterized in that** the peripheral zone of a plurality of targeted zones comprises a free zone (Zf) forming a ring delimited by a circle (611) of diameter equal to eleven centimetres and by the circle (521) of diameter equal to fourteen centimetres, in the free zone, no reflective coating covers or forms a part of the internal surface of the textile base.

5. Garment (1) according to the preceding claim, **characterized in that** the peripheral zone comprises an intermediate sector covering a ring delimited by an internal diameter (51) equal to the diameter (41) of said disc (4) and by an external diameter (611) of a circle (61) equal to eleven centimetres and in which a reflective coating (3) is present and covers or forms a part of the internal surface of the textile base, at the internal surface, in a proportion of less than 50% of the internal surface of the ring forming the intermediate sector.

6. Garment (1) according to one of the preceding claims, **characterized in that** the layer of reflective coating (3) entirely covers the textile base (2) over at least a dense part (Zd) of the targeted zone (Zc), this dense part being defined by a disc (7) of which the diameter (71) is at least equal to 1.5 centimetres.

7. Garment (1) according to one of the preceding claims, **characterized in that** a plurality of targeted zones are connected by connection zones provided with reflective coating.

8. Garment (1) according to one of the preceding claims, **characterized in that** a plurality of reflective coatings are associated with the textile base by printing, adhesive bonding, coating, weaving, knitting or embroidery.

9. Garment (1) according to one of the preceding claims, **characterized in that** the reflective coating is provided on separate pieces intended to be removably joined to the textile base.

10. Garment (1) according to one of the preceding claims, **characterized in that** the textile base is a knitted, woven or nonwoven textile.

11. Garment (1) according to one of the preceding claims, **characterized in that** the reflective coating (3) forms a spiral or a combination of spirals.

12. Garment (1) according to one of the preceding claims, **characterized in that** the reflective coating (3) forms an overlayer of which the thickness is less than one millimetre.

13. Garment (1) according to one of the preceding claims, **characterized in that** the reflective coating (3) comprises at least one of the following mineral metal oxides: Al₂O₃, Fe₂O₃, TiO₂, Cr₂O₃, SiO₂, MgO, ZrO₂, MnO₂, Co₂O₃, Y₂O₃ and mixtures thereof.
